(19) Europäisches Patentamt
European Patent Office
Office européen des brevets

(11) EP 0 869 952 B1

(12) FASCICULE DE BREVET EUROPEEN

(45) Date de publication et mention de la délivrance du brevet: **19.09.2001 Bulletin 2001/38**

(21) Numéro de dépôt: **96941108.1**

(22) Date de dépôt: **04.12.1996**

(51) Int Cl.[7]: **C07D 319/06**, C07D 405/12

(86) Numéro de dépôt international: **PCT/FR96/01926**

(87) Numéro de publication internationale: **WO 97/20836 (12.06.1997 Gazette 1997/25)**

# (54) DERIVES DE 5-NAPHTALEN-1-Y1-1,3-DIOXANES, LEUR PREPARATION ET LEUR APPLICATION EN THERAPEUTIQUE

DERIVATE VON 5-NAPHTHALIN-1-YL-1,3-DIOXANEN, IHRE HERSTELLUNG UND THERAPEUTISCHE ANWENDUNG

5-NAPHTHALEN-1-YL-1,3-DIOXANE DERIVATIVES, PREPARATION AND THERAPEUTICAL USE THEREOF

(84) Etats contractants désignés:
**AT BE CH DE DK ES FI FR GB GR IE IT LI LU NL PT SE**
Etats d'extension désignés:
**AL LT LV RO SI**

(30) Priorité: **06.12.1995 FR 9514394**

(43) Date de publication de la demande: **14.10.1998 Bulletin 1998/42**

(73) Titulaire: **SANOFI-SYNTHELABO**
**75013 Paris (FR)**

(72) Inventeurs:
- **DARGAZANLI, Gihad**
  **F-94240 L'Hay-les-Roses (FR)**
- **EVANNO, Yannick**
  **F-78830 Bullion (FR)**
- **FROST, Jonathan**
  **F-91320 Wissous (FR)**
- **LARDENOIS, Patrick**
  **F-92340 Bourg-la-Reine (FR)**
- **SEVRIN, Mireille**
  **F-75014 Paris (FR)**
- **GEORGE, Pascal**
  **F-78730 Saint-Arnoult-en-Yvelines (FR)**

(74) Mandataire: **Ludwig, Jacques et al**
**Sanofi-Synthélabo**
**Service Brevets**
**174, avenue de France**
**75013 Paris (FR)**

(56) Documents cités:
**EP-A- 0 461 958**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 869 952 B1

Printed by Jouve, 75001 PARIS (FR)

## Description

Dérivés de 5-naphtalén-1-yl-1,3-dioxanes, leur préparation et leur application en thérapeutique.

La présente invention a pour objet des composés de formule générale (I)

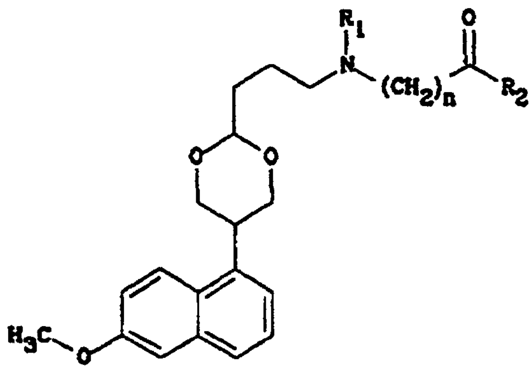

(I)

dans laquelle

$R_1$ représente un atome d'hydrogène ou un groupe ($C_1$-$C_4$)-alkyle, ($C_3$-$C_6$)cycloalkylméthyle ou phényl($C_1$-$C_3$) alkyle éventuellement substitué sur le noyau phényle par un ou plusieurs atomes ou groupes choisis parmi les halogènes et les groupes méthyle, trifluorométhyle, méthoxy et cyano,

$R_2$ représente un groupe hydroxy ou ($C_1$-$C_4$)alcoxy ou un groupe de formule générale $NR_3R_4$ dans laquelle $R_3$ et $R_4$, indépendamment l'un de l'autre, représentent chacun un atome d'hydrogène, un groupe ($C_1$-$C_4$)alkyle linéaire ou éventuellement ramifié, un groupe ($C_3$-$C_6$)cycloalkyle, un groupe ($C_3$-$C_6$)cycloalkylméthyle, un groupe phényle, un groupe phénylméthyle ou un groupe pyridinyle, ou bien encore $R_3$ et $R_4$ forment ensemble, et avec l'atome d'azote qui les porte, un cycle pyrrolidine ou pipéridine, et

n représente le nombre 1, 2 ou 3.

Les composés de l'invention peuvent exister sous forme de stéréoisomères *cis* ou *trans* ou de mélanges de tels isomères ; il peuvent également exister à l'état de bases libres ou de sels d'addition à des acides.

Les composés préférés sont ceux dans la formule générale (I) desquels $R_1$ représente un groupe méthyle, éthyle ou phénylméthyle éventuellement substitué sur le noyau phényle, $R_2$ représente un groupe amino ou ($C_1$-$C_4$) alkylamino, et n est égal à 1 ; parmi ces derniers, le composé dans la formule duquel $R_1$ représente un groupe phénylméthyle et $R_2$ représente un groupe amino est particulièrement intéressant.

Les composés selon l'invention peuvent être préparés par divers procédés.

Selon une première variante, on peut faire réagir une amine de formule générale (II)

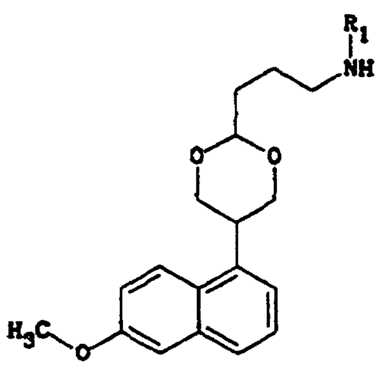

(II)

dans laquelle $R_1$ représente un atome d'hydrogène ou un groupe alkyle, avec un alcanoate ω-halogéné de formule générale (III)

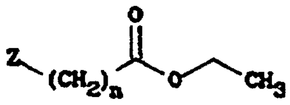

(III)

dans laquelle Z représente un atome de chlore ou de brome et n est tel que défini ci-dessus ; on obtient ainsi un composé de formule générale (I) dans laquelle $R_1$ représente un atome d'hydrogène ou un groupe alkyle et $R_2$ représente un groupe éthoxy. Si on le désire, on peut ensuite saponifier le composé ainsi obtenu, pour le transformer en l'acide correspondant, ou bien on peut le faire réagir avec une amine de formule générale $HNR_3R_4$, dans laquelle $R_3$ et $R_4$ sont tels que définis ci-dessus, pour le transformer en amide. Les conditions de ces réaction sont classiques et sont bien connues de l'homme du métier.

Selon une deuxième variante, on peut obtenir les amides de formule générale (I) dans laquelle $R_1$ représente un atome d'hydrogène ou un groupe alkyle en faisant réagir directement l'amine de formule générale (II) avec un alcanamide ω-halogéné de formule générale (IV)

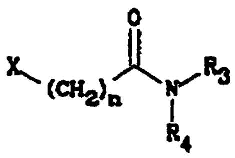

(IV)

dans laquelle X représente un atome de chlore ou de brome et $R_3$, $R_4$ et n sont tels que définis ci-dessus. Les conditions de cette réaction sont bien connues de l'homme du métier.

Selon une troisième variante, on peut obtenir les amides de formule générale (I) dans laquelle $R_1$ représente un atome d'hydrogène ou un groupe alkyle et n=2 en faisant réagir l'amine de formule générale (II) avec un propénamide de formule générale (V)

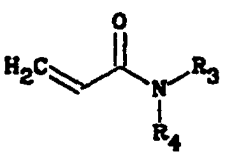

(V)

dans laquelle $R_3$ et $R_4$ sont tels que définis ci-dessus. Les conditions de cette réaction de Michael sont bien connues.

Enfin, et selon une quatrième variante, on peut préparer les composés de formule générale (I) dans laquelle $R_1$ ne représente pas un atome d'hydrogène par alkylation du composé correspondant dans la formule duquel $R_1$ représente un atome d'hydrogène, dans un solvant polaire aprotique, par exemple l'acétonitrile, en présence d'une base, par exemple de carbonate de potassium.

Les amines de départ de formule générale (II) dans laquelle $R_1$ représente un groupe alkyle peuvent être obtenues par réduction des alcanamides correspondants, décrits dans la demande de brevet EP-461958 ; les amines de départ de formule générale. (II) dans laquelle $R_1$ représente un atome d'hydrogène peuvent être obtenues par réaction du 2-(6-méthoxynaphtalén-1-yl)propane-1,3-diol avec la 4,4-diéthoxybutanamine, comme décrit dans ladite demande de brevet.

Les composés de départ de formules générales (III), (IV) et (V) sont disponibles dans le commerce ou bien peuvent être obtenus par des méthodes connues.

Les exemples qui vont suivre illustrent la préparation de quelques composés de l'invention. Les microanalyses élémentaires, et les spectres I.R. et R.M.N. confirment les structures des composés obtenus.

Les numéros indiqués entre parenthèses dans les titres des exemples correspondent à ceux de la 1ère colonne du tableau 1 donné plus loin.

Dans les noms des composés, le tiret "-" fait partie du nom, et le tiret "_" ne sert que pour la coupure en fin de ligne ; il est à supprimer en l'absence de coupure, et ne doit être remplacé ni par un tiret normal ni par un espace.

Exemple 1 (Composé N°1).

2-[[3-[5-(6-Méthoxynaphtalén-1-yl)-1,3-dioxan-2-yl]propyl]_ amino]acétamide.

1.1. Chlorhydrate de 5-(6-méthoxynaphtalén-1-yl)-1,3-dioxane-2-propanamine.

Dans un ballon de 1 1 contenant 300 ml de toluène on introduit 7,56 g (32,5 mmoles) de 2-(6-méthoxynaphtalén-1-yl)pro_ pane-1,3-diol, 6,8 g (42,1 mmoles) de 4,4-diéthoxybutanamine, puis 70 ml d'éther chlorhydrique, et on chauffe le mélange au reflux pendant 2 h.
On le refroidit, on collecte le précipité par filtration, et on le rince à l'éther diéthylique.
On obtient 10,2 g de chlorhydrate brut sous forme de solide beige.
Point de fusion : 224-226°C.

1.2. 2-[[3-[5-(6-Méthoxynaphtalén-1-yl)-1,3-dioxan-2-yl]propyl]amino]acétamide.

Dans un ballon de 100 ml contenant 30 ml d'acétonitrile on introduit 1,2 g (4 mmoles) de 5-(6-méthoxynaphtalén-1-yl)-1,3-dioxane-2-propanamine, 0,8 g (5,8 mmoles) de carbonate de potassium et 0,4 g (4,2 mmoles) de chloroacétamide, et on chauffe le mélange à 80°C pendant 3 h.
On le laisse refroidir, on ajoute 50 ml d'eau, et on extrait avec deux fois 50 ml d'acétate d'éthyle. On lave la phase organique à l'eau, on la sèche sur sulfate de magnésium, on évapore le solvant sous pression réduite et on purifie le résidu par chromatographie sur colonne de gel de silice en éluant avec un mélange 95/5 de dichlorométhane et de méthanol.
On obtient 0,4 g (1,1 mmole) de solide blanc qu'on recristallise dans l'éthanol.
Point de fusion : 148-150°C.

Exemple 2 (Composé N°6).

2-[Ethyl[3-[5-(6-méthoxynaphtalén-1-yl)-1,3-dioxan-2-yl]pro_ pyl]amino]acétamide.

2.1. Chlorhydrate de N-éthyl-5-(6-méthoxynaphtalén-1-yl)-1,3-dioxane-2-propanamine.

Dans un ballon de 500 ml contenant une suspension de 1,66 g (43,6 mmoles) d'hydrure de lithium et d'aluminium dans 50 ml de tétrahydrofurane, chauffée au reflux, on ajoute une suspension de 10 g (29,1 mmoles) de *N*-[3-[5-(6-méthoxynaphtalén-1-yl)-1,3-dioxan-2-yl]propyl]acétamide dans 75 ml de tétra_ hydrofurane, et on maintient le reflux et l'agitation pendant 3 h.
On refroidit le mélange, on l'hydrolyse en ajoutant 10,5 ml d'une solution 1 M de tartrate de sodium et de potassium, on l'agite pendant 12 h, on sépare le solide par filtration, en lavant ce dernier avec du tétrahydrofurane, et on concentre le filtrat à sec sous pression réduite.
On obtient 10,27 g de produit huileux dont on prélève 0,5 g pour former le chlorhydrate dans l'éthanol.
Point de fusion : 165°C (décomposition).

2.2. 2-[Ethyl[3-[5-(6-méthoxynaphtalén-1-yl)-1,3-dioxan-2-yl]propyl]amino]acétamide.

Dans un ballon de 100 ml contenant 40 ml de *N,N*-diméthylformamide on introduit 3 g (8,2 mmoles) de chlorhydrate de *N*-éthyl-5-(6-méthoxynaphtalén-1-yl)-1,3-dioxane-2-propanamine, 5,6 g (40,5 mmoles) de carbonate de potassium et 0,9 g (9,6 mmoles) de chloroacétamide, et on chauffe le mélange à 80°C pendant 4 h.
On le laisse refroidir, on ajoute 70 ml d'eau et on l'extrait avec deux fois 100 ml d'acétate d'éthyle. On lave la phase organique à l'eau, on la sèche sur sulfate de magnésium, on la filtre, on évapore le solvant sous pression réduite et on purifie le résidu par chromatographie sur colonne de gel de silice en éluant avec un mélange de dichlorométhane et de méthanol 98/2.
On obtient 2,6 g (6,7 mmoles) de solide blanc qu'on recristallise dans un mélange d'éther isopropylique et de dichloro_ méthane.
Point de fusion : 120-121°C.

Exemple 3 (Composé N°10) .

(*E*)-But-2-ènedioate de 2-[éthyl[3-[5-(6-méthoxynaphtalén-1-yl) -1,3-dioxan-2-yl] propyl] amino]-*N*-pyridin-2-ylacétamide (1:1).

Dans un ballon de 100 ml contenant 25 ml d'acétonitrile on introduit 1,4 g (4,24 mmoles) de *N*-éthyl-5-(6-méthoxynaphtalén-1-yl)-1,3-dioxane-2-propanamine, 0,6 g (4,3 mmoles) de carbonate de potassium et 0,72 g (4,2 mmoles) de 2-chloro-*N*-pyridin-2-ylacétamide, et on agite le mélange à 25°C pendant 12 h.
On ajoute 50 ml d'eau et on extrait avec deux fois 50 ml d'acétate d'éthyle. On lave la phase organique à l'eau, on la sèche sur sulfate de magnésium, on la filtre, on évapore le solvant sous pression réduite et on purifie le résidu par chromatographie sur colonne de gel de silice en éluant avec un mélange d'éther de pétrole et d'acétate d'éthyle 70/30.
On obtient 1,46 g (3,15 mmoles) de composé qu'on cristallise sous forme de fumarate dans l'éther isopropylique.
Point de fusion : 60-67°C.

Exemple 4 (Composé N°17).

Ethanedioate de 3-[éthyl[3-[5-(6-méthoxynaphtalén-1-yl)-1,3-dioxan-2-yl]propyl]amino]propanamide (1:1).

Dans un ballon de 100 ml contenant 20 ml d'acétonitrile on introduit 1,3 g (4 mmoles) de N-éthyl-5-(6-méthoxynaphtalén-1-yl)-1,3-dioxane-2-propanamine et 0,43 g (6 mmoles) d'acrylamide, et on chauffe le mélange au reflux pendant 8 h.
On ajoute à nouveau 0,3 g (4 mmoles) d'acrylamide et on maintient le reflux pendant 8 h supplémentaires.
On refroidit le mélange, on le concentre à sec sous pression réduite, on reprend le résidu avec 50 ml d'eau et on l'extrait avec deux fois 50 ml d'acétate d'éthyle. On lave la phase organique à l'eau, on la sèche sur sulfate de magnésium, on la filtre, on évapore le solvant sous pression réduite et on purifie le résidu par chromatographie sur colonne de gel de silice en éluant avec un mélange de dichlo_ rométhane et de méthanol 97/3.
On obtient 0,98 g de composé que l'on fait cristalliser sous forme d'oxalate dans le propan-2-ol.
Point de fusion : 125°C (décomposition).

Exemple 5 (Composé N°7).

Ethanedioate de 2-[éthyl[3-[5-(6-méthoxynaphtalén-1-yl)-1,3-dioxan-2-yl]propyl]amino]-*N*-méthylacétamide (1:1).

5.1. 2-[Ethyl[3-[5-(6-méthoxynaphtalén-1-yl)-1,3-dioxan-2-yl]propyl]amino]acétate d'éthyle.

Dans un ballon de 100 ml contenant 21 ml d'acétonitrile on introduit 1,4 g (4,24 mmoles) de *N*-éthyl-5-(6-méthoxynaphtalén-1-yl)-1,3-dioxane-2-propanamine, 1,75 g (12,7 mmoles) de carbonate de potassium et 0,5 ml (4,45 mmoles) de bromo_ acétate d'éthyle, et on chauffe le mélange au reflux pendant 2 h.
On le refroidit, on le concentre à sec sous pression réduite, on reprend le résidu avec 50 ml d'eau et on extrait avec deux fois 50 ml d'acétate d'éthyle. On lave la phase organique à l'eau, on la sèche sur sulfate de magnésium, on la filtre et on évapore le solvant sous pression réduite. On purifie le résidu par chromatographie sur colonne de gel de silice en éluant avec un mélange de dichlorométhane et de méthanol 99/1 et on obtient 1,17 g (3 mmoles) de composé sous forme d'huile incolore. On en prépare l'oxalate de manière classique;
Point de fusion : 124-127°C.

5.2. Ethanedioate de 2-[éthyl[3-[5-(6-méthoxynaphtalén-1-yl)-1,3-dioxan-2-yl]propyl]amino]-*N*-méthylacétamide (1:1).

Dans un réacteur de 250 ml contenant 11 ml d'une solution de méthylamine à 33% dans l'éthanol on introduit 1,15 g (3 mmoles) de 2-[éthyl[3-[5-(6-méthoxynaphtalén-1-yl)-1,3-dioxan-2-yl]propyl]amino]acétate d'éthyle et on chauffe le mélange à 50°C sous scellé pendant 5 j.
On le refroidit, on le concentre à sec sous pression réduite, on reprend le résidu avec 50 ml d'eau et on l'extrait avec deux fois 50 ml d'acétate d'éthyle. On lave la phase organique à l'eau, on la sèche sur sulfate de magnésium, on la filtre et on évapore le solvant sous pression réduite. On obtient 1,16 g (2,89 mmoles) de composé sous forme d'huile jaune que l'on cristallise sous forme d'oxalate dans le propan-2-ol.
Point de fusion : 140°C (décomposition).

Exemple 6 (Composé N°18).

Ethanedioate de 4-[éthyl[3-[5-(6-méthoxynaphtalén-l-yl)-1,3-dioxan-2-yl]propyl]amino]butanoate d'éthyle (1:1).

Dans un ballon de 100 ml contenant 50 ml de *N*,*N*-diméthylformamide on introduit 7,31 g (20 mmoles) de chlorhydrate de *N*-éthyl-5-(6-méthoxynaphtalén-1-yl)-1,3-dioxane-2-propanamine, 2,76 g (20 mmoles) de carbonate de potassium et 3,9 g (20 mmoles) de bromobutanoate d'éthyle, et on chauffe le mélange à 80°C pendant 7 h.
On le laisse refroidir, on le concentre à sec sous pression réduite, on reprend le résidu avec 70 ml d'eau, on extrait avec deux fois 100 ml d'acétate d'éthyle, on lave la phase organique à l'eau, on la sèche sur sulfate de magnésium, on la filtre, on évapore le solvant sous pression réduite et on purifie le résidu par chromatographie sur colonne de gel de silice en éluant avec un mélange 90/10 de dichlorométhane et de méthanol.
On obtient 6,1 g (13,7 mmoles) de composé huileux que l'on cristallise sous forme d'oxalate dans l'acétate d'éthyle.
Point de fusion : 132-135°C.

Exemple 7 (Composé N°15) .

Chlorhydrate de 2-[[3-[5-(6-méthoxynaphtalén-1-yl)-1,3-dioxan-2-yl]propyl](phénylméthyl)amino]acétamide.

Dans un ballon de 25 ml contenant 8 ml d'acétonitrile on introduit 0,5 g (1,39 mmole) de 2-[[3-[5-(6-méthoxynaphtalén-1-yl)-1,3-dioxan-2-yl]propyl]amino]acétamide. 0,29 g (2,1 mmoles) de carbonate de potassium et 0,18 ml (1,5 mmole) de bromure de benzyle, et on chauffe le mélange au reflux pendant 2 h.
On le laisse refroidir, on ajoute 15 ml d'eau, on extrait avec deux fois 15 ml d'acétate d'éthyle, on lave la phase organique à l'eau , on la sèche sur sulfate de magnésium, on la filtre, on évapore le solvant sous pression réduite et on purifie le résidu par chromatographie sur colonne de gel de silice en éluant avec un mélange 98/2 de dichlorométhane et de méthanol.
On obtient 0,24 g (0,53 mmole) de composé sous forme d'huile que l'on cristallise sous forme de chlorhydrate dans l'éther isopropylique.
Point de fusion : 218-220°C.

Exemple 8 (Composé N° 21).

Ethanedioate de 2-[éthyl[3-[5-(6-méthoxynaphtalén-1-yl)-1,3-dioxan-2-yl]propyl]amino]-*N*-(1-méthyléthyl)acétamide (1:1).

Dans un ballon de 250 ml contenant 20 ml de dioxane on introduit, sous atmosphère inerte, 0,23 ml (2,73 mmoles) de 1-méthyléthylamine, 0,38 ml (2,73 mmoles) de triéthylamine puis une solution de 0,22 ml (2,73 mmoles) de chlorure de chloroacétyle dans 10 ml de dioxane, et on laisse le mélange sous agitation pendant 15 h.
On ajoute 30 ml d'eau, puis 1 g (7,23 mmoles) de carbonate de potassium, puis 1 g (2,73 mmoles) de chlorhydrate de *N*-éthyl-5-(6-méthoxynaphtalén-1-yl)-1,3-dioxane-2-propanamine et on chauffe le mélange à 80°C pendant 7 h.
On le refroidit, on ajoute 80 ml d'eau, on extrait avec deux fois 50 ml d'acétate d'éthyle, on lave la phase organique à l'eau, on la sèche sur sulfate de magnésium, on la fitre et on évapore le solvant sous pression réduite. On purifie le produit obtenu par chromatographie sur colonne de gel de silice en éluant avec un mélange 95/5 de dichlorométhane et de méthanol et on obtient 0,8 g (1,86 mmole) de composé sous forme d'huile incolore.
On en prépare l'oxalate de manière classique.
Point de fusion : 130-131°C.

Exemple 9 (Composé N°37).

Chlorhydrate de 2-[[(2-fluorophényl)méthyl][3-[5-(6-méthoxy_ naphtalén-1-yl)-1,3-dioxan-2-yl]propyl]amino]acétamide (1:1).

Dans un ballon de 100 ml contenant 20 ml d'acétonitrile on introduit 1 g (2,79 mmoles) de 2-[[3-[5-(6-méthoxynaphtalén-1-yl)-1,3-dioxan-2-yl]propyl]amino] acétamide, 0,8 g (5,78 mmoles) de carbonate de potassium et 0,5 ml (4,2 mmoles) de 1-(chlorométhyl)-2-fluorobenzène, et on chauffe le mélange au reflux pendant 6 h.
On le laisse refroidir, on ajoute 30 ml d'eau, et on extrait avec deux fois 20 ml d'acétate d'éthyle. On lave la phase organique à l'eau, on la sèche sur sulfate de magnésium, on la filtre et on évapore le solvant sous pression réduite. On purifie le produit obtenu par chromatographie sur colonne de gel de silice en éluant avec un mélange 98/2 de dichloro_ méthane et de méthanol.

On obtient 0,34 g (0,73 mmole) de composé sous forme d'huile incolore, que l'on fait cristalliser sous forme de chlorhydrate dans le propan-2-ol.
Point de fusion : 200-202°C.

Exemple 10 (Composé N°30).

(*E*)-2-butènedioate de 2-[(cyclopropylméthyl)[3-[5-(6-méthoxynaphtalén-1-yl)-1,3-dioxan-2-yl]propyl]amino]acétamide (1:1).

Dans un ballon de 250 ml contenant 15 ml de *N,N*-diméthylformamide on introduit 1 g (2,79 mmoles) de 2-[[3-[5-(6-méthoxynaphtalén-1-yl)-1,3-dioxan-2-yl]propyl]amino]acétamide, 0,8 g (5,78 mmoles) de carbonate de potassium, 0,38 g (4,2 mmoles) de (chlorométhyl)cyclopropane et une quantité catalytique d'iodure de sodium, et on chauffe le mélange à 100°C pendant 20 h.
On le laisse refroidir, on évapore le solvant sous pression réduite, on reprend le résidu avec du dichlorométhane, on sépare l'insoluble par filtration, on concentre le filtrat à sec sous pression réduite, et on purifie le produit obtenu par chromatographie sur colonne de gel de silice en éluant avec un mélange 9/1 de dichlorométhane et de méthanol.
On obtient 0,60 g (1,45 mmole) de composé sous forme d'huile incolore, que l'on fait cristalliser sous forme de fumarate dans le propan-2-ol.
Point de fusion : 149-150°C.

Exemple 11 (Composé N°41).

2-[[3-[5-(6-méthoxynaphtalén-1-yl)-1,3-dioxan-2-yl]propyl]_ (phénylméthyl)amino]acétate d'éthyle.

11.1 2-[[3-[5-(6-méthoxynaphtalén-1-yl)-1,3-dioxan-2-yl]pro_ pyl]amino]acétate d'éthyle.

Dans un ballon de 500 ml contenant 110 ml de *N,N*-diméthylformamide on introduit 6,64 g (22 mmoles) de 5-(6-méthoxynaphtalén-1-yl)-1,3-dioxan-2-propanamine, 3 g (22 mmoles) de carbonate de potassium et 3,54 ml (33 mmoles) de chloroacétate d'éthyle, et on chauffe le mélange à 80°C pendant 20 min.
On le laisse refroidir, on évapore le solvant sous pression réduite, on reprend le résidu avec 50 ml d'eu et on extrait avec deux fois 50 ml d'acétate d'éthyle. On lave la phase organique à l'eau, on la sèche sur sulfate de magnésium, on la filtre, on concentre le filtrat à sec sous pression réduite et on purifie le produit obtenu par chromatographie sur colonne de gel de silice en éluant avec un mélange 98/2 de dichlorométhane et de méthanol.
On obtient 3,52 g (9,08 mmoles) de composé sous forme d'huile incolore qu'on utilise tel quel dans l'étape suivante.

11.2. 2-[[3-[5-(6-méthoxynaphtalén-1-yl)-1,3-dioxan-2-yl]propyl] (phénylméthyl)amino]acétate d'éthyle.

Dans un ballon de 250 ml contenant 45 ml d'acétonitrile on introduit 3,52 g (9,08 mmoles) de 2-[[3-[5-(6-méthoxynaph_ talén-1-yl)-1,3-dioxan-2-yl]propyl]amino]acétate d'éthyle, 1,2 g de carbonate de potassium et 1,05 ml (9,12 mmoles) de (chlorométhyl)benzène, et on chauffe le mélange à 80°C pendant 6 h.
On le laisse refroidir, on le concentre à sec sous pression réduite, on reprend le résidu avec 50 ml d'eau, on extrait avec deux fois 50 ml d'acétate d'éthyle, on lave la phase organique à l'eau, on la sèche sur sulfate de magnésium, on la filtre et on évapore le solvant sous pression réduite. On obtient 3,7 g de produit huileux dont on purifie 1 g par chromatographie sur colonne de gel de silice en éluant avec un mélange 99/1 de dichlorométhane et de méthanol.
On obtient 0,5 (2,09 mmoles) de composé sous forme d'huile incolore qui cristallise dans l'éther diisopropylique.
Point de fusion : 58-60°C.

Exemple 12 (Composé N°39).

Chlorhydrate de 2-[[3-[5-(6-méthoxynaphtalén-1-yl)-1,3-dioxan-2-yl]propyl](phénylméthyl)amino]-*N*-méthylacétamide (1:1).

Dans un réacteur de 250 ml contenant 8 ml d'une solution de méthylamine à 33% dans l'éthanol on introduit 1 g (2,15 mmoles) de 2-[[3-[5-(6-méthoxynaphtalén-1-yl)-1,3-dioxan-2-yl]propyl] (phénylméthyl)amino]acétate d'éthyle et on chauffe le mélange à 50°C sous scellé pendant 3 j.
On le laisse refroidir, on le concentre à sec sous pression réduite, on reprend le résidu avec 50 ml d'eau, on l'extrait avec deux fois 50 ml d'acétate d'éthyle, on lave la phase organique à l'eau, on la sèche sur sulfate de magnésium, on la filtre, on évapore le solvant sous pression réduite. On purifie le produit obtenu par chromatographie sur colonne de

gel de silice en éluant avec un mélange 98/2 de dichloro_ méthane et de méthanol.
On obtient 0,51 g (1,1 mmole) de composé sous forme d'une huile incolore qu'on fait cristalliser sous forme de chlorhydrate dans l'éther diisopropylique.
Point de fusion : 98-100°C.

Le tableau qui suit illustre les structures chimiques et les propriétés physiques de quelques composés selon l'invention.

Tableau

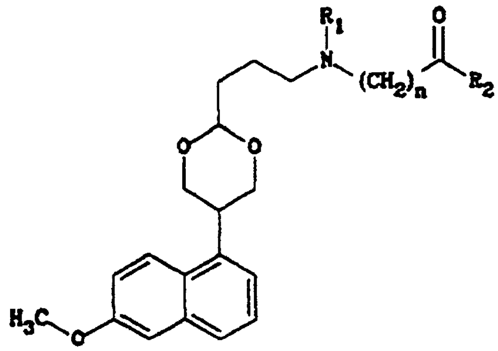

(I)

| N° | $R_1$ | $R_2$ | n | Sel | F(°C) ou $n_D^{20}$ |
|---|---|---|---|---|---|
| 1 | H | $NH_2$ | 1 | - | 148-150 |
| 2 | $CH_3$ | $OCH_2CH_3$ | 1 | ox. (1:1) | 127-128 |
| 3 | $CH_3$ | $NH_2$ | 1 | - | 146-147 |
| 4 | $CH_3$ | $NHCH_3$ | 1 | -<br>ox. (1:1) | 106-107<br>169-170 |
| 5 | $CH_2CH_3$ | $OCH_2CH_3$ | 1 | ox. (1:1) | 124-127 |
| 6 | $CH_2CH_3$ | $NH_2$ | 1 | - | 120-121 |
| 7 | $CH_2CH_3$ | $NHCH_3$ | 1 | ox. (1:1) | 140 (d) |
| 8 | $CH_2CH_3$ | $NHC_6H_5$ | 1 | ox. (1:1)<br>fum. (1:1) | 168-169<br>70-72 |
| 9 | $CH_2CH_3$ | $NHCH_2C_6H_5$ | 1 | ox. (1:1) | 145-146 |
| 10 | $CH_2CH_3$ | $NH-2-NC_5H_4$ | 1 | fum. (1:1) | 60-67 |
| 11 | $CH_2CH_3$ | $NHC_6H_{11}$ | 1 | - | 102-103 |
| 12 | $CH_2CH_3$ | $NC_5H_{10}$ | 1 | - | $n_D^{20}$=1,571 |
| 13 | $CH_2CH_3$ | $NC_4H_8$ | 1 | - | $n_D^{20}$=1,573 |
| 14 | $CH_2CH_3$ | $NHCH_2CH(CH_3)_2$ | 1 | ox. (1:1) | 136-137 |

| N° | $R_1$ | $R_2$ | n | Sel | F(°C) ou $n_D^{20}$ |
|---|---|---|---|---|---|
| 15 | $CH_2C_6H_5$ | $NH_2$ | 1 | HCl (1:1) | 218-220 |
| 16 | H | $NH_2$ | 2 | - | 122 (d) |
| 17 | $CH_2CH_3$ | $NH_2$ | 2 | ox. (1:1) | 125 (d) |
| 18 | $CH_2CH_3$ | $OCH_2CH_3$ | 3 | ox. (1:1) | 132-135 |
| 19 | $CH_2CH_3$ | $NH_2$ | 3 | - | 89-90 |
| 20 | $CH_2CH_3$ | $NHCH_3$ | 3 | - | $n_D^{20}$=1,571 |
| 21 | $CH_2CH_3$ | $NHCH(CH_3)_2$ | 1 | ox. (1:1) | 130-131 |
| 22 | $CH_2CH_3$ | $NHCH_2C_3H_5$ | 1 | ox. (1:1) | 126-127 |
| 23 | $CH_3$ | $NH_2$ | 2 | ox. (1:1) | 137 (d) |
| 24 | $CH_2CH_3$ | OH | 3 | - | 50-60 |
| 25 | $CH_2C_6H_4$-4-$CH_3$ | $NH_2$ | 1 | HCl (1:1) | 194 (d) |
| 26 | $CH_2C_6H_4$-3-$CH_3$ | $NH_2$ | 1 | - | 132-133 |
| 27 | $CH_2C_6H_4$-4-$OCH_3$ | $NH_2$ | 1 | HCl (1,1:1) | 160 (d) |
| 28 | $CH_2C_6H_4$-3-Cl | $NH_2$ | 1 | HCl (1:1) | 178 (d) |
| 29 | $CH_2C_6H_4$-4-Br | $NH_2$ | 1 | HCl (1,2:1) | 189-191 |
| 30 | $CH_2C_3H_5$ | $NH_2$ | 1 | fum. (1:1) | 149-150 |
| 31 | $CH_2C_6H_4$-3-$CF_3$ | $NH_2$ | 1 | fum. (1:1) | 168-169 |
| 32 | $CH_2C_6H_4$-4-Cl | $NH_2$ | 1 | HCl (1,1:1) | 160-162 |
| 33 | $CH_2C_6H_{11}$ | $NH_2$ | 1 | HCl (1:1) | 163-165 |
| 34 | $CH_2C_6H_4$-2-Cl | $NH_2$ | 1 | - | 117-118 |
| 35 | $CH_2C_6H_4$-3-$OCH_3$ | $NH_2$ | 1 | - | 107-108 |
| 36 | $CH_2C_6H_4$-3-F | $NH_2$ | 1 | HCl (1:1) | 160-162 |

| N° | $R_1$ | $R_2$ | n | Sel | F(°C) ou $n_D^{20}$ |
|---|---|---|---|---|---|
| 37 | $CH_2C_6H_4$-2-F | $NH_2$ | 1 | HCl (1:1) | 200-202 |
| 38 | $(CH_2)_2C_6H_5$ | $NH_2$ | 1 | HCl (1:1) | 55 (d) |
| 39 | $CH_2C_6H_5$ | $NHCH_3$ | 1 | HCl (1:1) | 98-100 |
| 40 | $CH_2C_6H_4$-2-$CH_3$ | $NH_2$ | 1 | HCl (0,7:1) | 187-189 |
| 41 | $CH_2C_6H_5$ | $OCH_2CH_3$ | 1 | - | 58-60 |
| 42 | $CH_2C_6H_4$-4-F | $NH_2$ | 1 | HCl (1:1) | 138-140 |
| 43 | $CH_2C_6H_5$ | $NHCH_2CH_3$ | 1 | HCl (1:1) | 70-72 |
| 44 | $CH_2C_6H_4$-2-$OCH_3$ | $NH_2$ | 1 | fum. (1:1) | 173-174 |
| 45 | $(CH_2)_3C_6H_5$ | $NH_2$ | 1 | HCl (1,1:1) | 150 (d) |
| 46 | $CH_2C_6H_4$-3-CN | $NH_2$ | 1 | fum. (1,1:1) | 163-165 |

Dans les colonnes "$R_1$" et "$R_2$", $C_3H_5$ représente un groupe cyclopropyle, $C_6H_{11}$ représente un groupe cyclohexyle, $C_6H_5$ représente un groupe phényle, $C_6H_4$-p-Y représente un groupe phényle portant un susbtituant Y en position p, 2-$NC_5H_4$ représente un groupe pyridin-2-yle, $NC_4H_8$ représente un groupe pyrrolidin-1-yle, $NC_5H_{10}$ représente un groupe pipéridin-1-yle.

Dans la colonne "Sel", "-" désigne un composé à l'état de base, "ox." désigne un oxalate (ou éthanedioate), "fum." désigne un fumarate (ou (*E*)-2-butènedioate) et "HCl" désigne un chlorhydrate ; le rapport molaire acide:base est indiqué entre parenthèses.

Dans la dernière colonne sont indiqués les points de fusion F(°C) ou les indices de réfraction $n_D^{20}$ ; "(d)" désigne un point de fusion avec décomposition.

Tous les composés sont des stéréoisomères *trans* ($^1$H RMN), excepté le composé N°24 qui est un mélange de stéréoisomères *cis* et *trans* à majorité *trans.*

Les composés selon l'invention ont fait l'objet d'essais pharmacologiques qui ont mis en évidence leur intérêt comme substances thérapeutiques.

Activité neuroprotectrice vis-à-vis de l'ischémie focale chez le rat.

L'activité neuroprotectrice des composés selon l'invention a été démontrée dans un modèle d'ischémie focale permanente provoquée par occlusion intraluminale de l'artère cérébrale moyenne chez le rat, selon une méthode analogue à celle décrite dans Stroke (1989) 20 84-91.

Sous anesthésie au methohexital sodique on ligature l'artère ptérygopalatine, l'artère carotidienne commune et l'artère carotidienne externe gauche et on introduit un fil de polyamide dans l'artère carotidienne interne sur une longueur d'environ 18 mm correspondant à la distance qui sépare la naissance de l'artère carotidienne interne de celle de l'artère cérébrale moyenne.

Les composés à étudier sont administrés après l'occlusion par voie intraveineuse.

24 h après l'occlusion de l'artère cérébrale moyenne on sacrifie les animaux et on prélève le cerveau.

On évalue le volume de l'infarctus cérébral à partir de la mesure de la surface de la nécrose sur 6 coupes coronales colorées par le chlorure de 2,3,5-triphényltétrazolium.

A titre d'exemple, les composés N°6 et N°15 du tableau qui précède réduisent significativement le volume de l'infarctus d'environ 31% et 50%, respectivement, à la dose de 3 mg/kg administrée par voie intraveineuse aux temps 10 min, 1

h 30 min, 3 h et 6 h après l'occlusion.

Activité vis-à-vis des convulsions toniques induites chez la souris par électrochoc supramaximal.

Le protocole de cet essai est décrit par E. A. Swinyard et J. H. Woodhead dans *Antiepileptic Drugs,* Raven Press, New York, 111-126 (1982).

10 min après administration intraveineuse du composé à tester, on note le nombre de souris présentant des convulsions toniques (extensions des pattes antérieures et postérieures), immédiatement après application d'un courant électrique (0,4 s, 60 mA, 50 Hz) à l'aide d'un appareil Apelex ETC UNIT 7801™. Les résultats sont exprimés par la $DA_{50}$, dose qui protège 50% des animaux, calculée selon la méthode de J. T. Lichtfield et F. Wilcoxon (J. *Pharm. Exp. Ther.*, 96, 99-113 (1949)), à l'aide du logiciel Probit™, à partir de 3 ou 4 doses administrées chacune à un groupe de 8 souris.

Les $DA_{50}$ des composés de l'invention se situent, dans cet essai, entre 0,5 et 10 mg/kg.

Les résultats des essais montrent que les composés selon l'invention ont des propriétés neuroprotectrices, et qu'ils peuvent donc être utilisés pour la préparation de médicaments utiles dans le traitement ou la prévention des désordres cérébrovasculaires d'origine ischémique ou hypoxique (infarctus cérébral, traumatisme crânien ou médullaires, arrêt cardiaque ou respiratoire, attaque ischémique transitoire, asphyxie périnatale), du glaucome, des maladies neurodégénératives progressives (démences séniles comme la maladie d'Alzheimer, démences vasculaires, maladie de Parkinson, maladie de Huntington, atrophie olivo-ponto-cérébelleuse, sclérose latérale amyotrophique, maladies neurodégénératives d'origine virale, etc), et dans la prévention des accidents ischémiques cérébraux associés à la chirurgie cardiaque et vasculaire et à la thérapie endovasculaire.

Du fait de leurs propriétés anticonvulsivantes ils peuvent également être utilisés dans le traitement de l'épilepsie. Enfin, le traitement d'autres affections, telles que les neuropathies, les douleurs neurogènes (par exemple liées aux neuropathies ou à la crise migraineuse), la spasticité neurologique et les dyskinésies, peut également être envisagé.

A cet effet ils peuvent être présentés sous toutes formes de compositions pharmaceutiques appropriées à l'administration entérale ou parentérale, telles que comprimés, dragées, gélules, capsules, suspensions ou solutions buvables ou injectables telles que sirops, ampoules, etc, associés à des excipients convenables, et dosés pour permettre une administration journalière de 0 à 1000 mg de substance active.

## Revendications

1. Composé, sous forme de stéréoisomère pur ou de mélange de stéréoisomères, répondant à la formule générale (I)

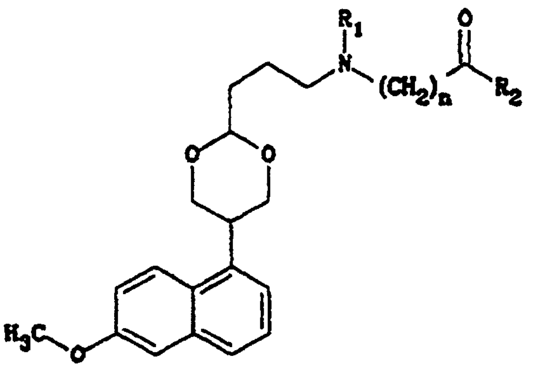

(I)

dans laquelle

- $R_1$ représente un atome d'hydrogène ou un groupe $(C_1-C_4)$-alkyle, $(C_3-C_6)$cycloalkylméthyle ou phényl$(C_1-C_3)$alkyle éventuellement substitué sur le noyau phényle par un ou plusieurs atomes ou groupes choisis parmi les halogènes et les groupes méthyle, trifluorométhyle, méthoxy et cyano,
- $R_2$ représente un groupe hydroxy ou $(C_1-C_4)$alcoxy ou un groupe de formule générale $NR_3R_4$ dans laquelle $R_3$ et $R_4$, indépendamment l'un de l'autre, représentent chacun un atome d'hydrogène, un groupe $(C_1-C_4)$alkyle linéaire ou éventuellement ramifié, un groupe $(C_3-C_6)$cycloalkyle, un groupe $(C_3-C_6)$cycloalkylméthyle, un groupe phényle, un groupe phénylméthyle ou un groupe pyridinyle, ou bien encore $R_3$ et $R_4$ forment ensemble, et avec l'atome d'azote qui les porte, un cycle pyrrolidine ou pipéridine, et
- n représente le nombre 1, 2 ou 3,

à l'état de base libre ou de sel d'addition à un acide.

**2.** Composé selon la revendication 1, **caractérisé en ce que** $R_1$ représente un groupe méthyle, éthyle ou phénylméthyle éventuellement substitué sur le noyau phényle, $R_2$ représente un groupe amino ou $(C_1-C_4)$alkylamino, et n est égal à 1.

**3.** Composé selon la revendication 1, **caractérisé en ce que** $R_1$ représente un groupe phénylméthyle, $R_2$ représente un groupe amino et n est égal à 1.

**4.** Composé selon la revendication 1, **caractérisé en ce que** $R_1$ représente un groupe méthyle, $R_2$ représente un groupe méthylamino et n est égal à 1.

**5.** Procédé de préparation de composés selon la revendication 1, **caractérisé en ce qu'**on fait réagir une amine de formule générale (II)

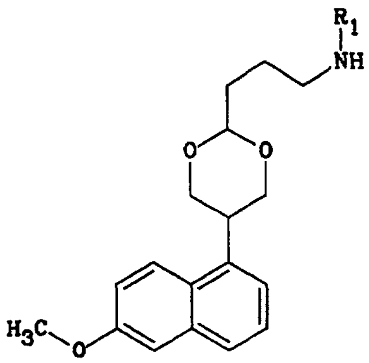

(II)

dans laquelle $R_1$ représente un atome d'hydrogène ou un groupe alkyle, avec un alcanoate ω-halogéné de formule générale (III)

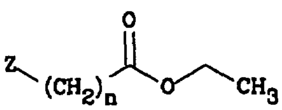

(III)

dans laquelle Z représente un atome de chlore ou de brome et n est tel que défini dans la revendication 1, pour obtenir un composé de formule générale (I) dans laquelle $R_1$ représente un atome d'hydrogène ou un groupe alkyle et $R_2$ représente un groupe éthoxy, puis, si on le désire, on saponifie le composé ainsi obtenu, pour le transformer en l'acide correspondant, ou bien on le fait réagir avec une amine de formule générale $HNR_3R_4$, dans laquelle $R_3$ et $R_4$ sont tels que définis dans la revendication 1, pour le transformer en amide.

**6.** Procédé de préparation de composés selon la revendication 1, **caractérisé en ce qu'**on fait réagir une amine de formule générale (II)

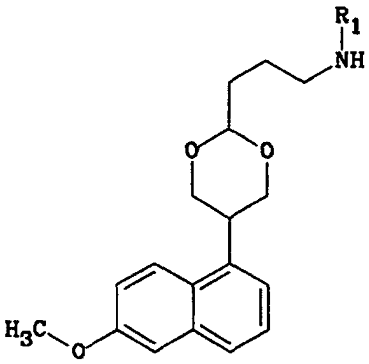

(II)

dans laquelle $R_1$ représente un atome d'hydrogène ou un groupe alkyle, avec un alcanamide ω-halogéné de formule générale (IV)

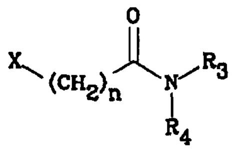

(IV)

dans laquelle X représente un atome de chlore ou de brome et $R_3$, $R_4$ et n sont tels que définis dans la revendication 1.

**7.** Procédé de préparation de composés selon la revendication 1, **caractérisé en ce qu'**on fait réagir une amine de formule générale (II)

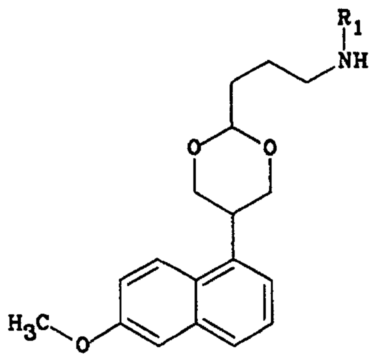

(II)

dans laquelle $R_1$ représente un atome d'hydrogène ou un groupe alkyle, avec un propénamide de formule générale (V)

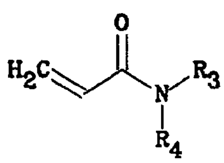

(V)

dans laquelle $R_3$ et $R_4$ sont tels que définis dans la revendication 1.

**8.** Procédé de préparation de composés selon la revendication 1, **caractérisé en ce qu'**on effectue une alkylation d'un composé de formule générale (I) dans laquelle $R_1$ représente un atome d'hydrogène.

**9.** Médicament **caractérisé en ce qu'**il consiste en un composé selon l'une des revendications 1 à 4.

**10.** Composition pharmaceutique, **caractérisée en ce qu'**elle contient un composé selon l'une des revendications 1 à 4, associé à un excipient.

## Patentansprüche

**1.** Verbindung in Form des reinen Stereoisomeren oder einer Mischung von Stereoisomeren der allgemeinen Formel (I)

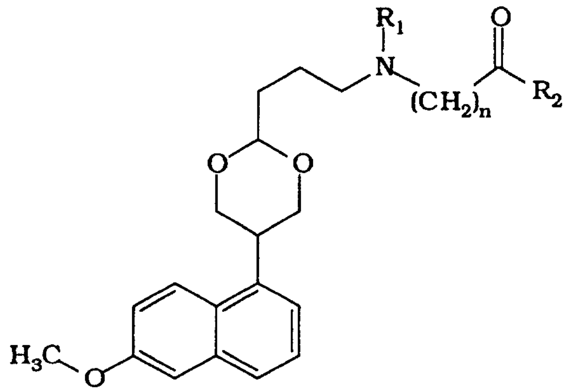

(I)

in der

$R_1$ ein Wasserstoffatom oder eine $(C_1-C_4)$-Alkylgruppe, $(C_3-C_6)$-Cycloalkylmethylgruppe oder Phenyl-$(C_1-C_3)$-alkylgruppe, die gegebenenfalls am Phenylkern durch ein oder mehrere Atome oder Gruppen ausgewählt aus Halogenen und Methyl-, Trifluormethyl-, Methoxy- und Cyanogruppen substituiert ist,

$R_2$ eine Hydroxygruppe oder $(C_1-C_4)$-Alkoxygruppe oder eine Gruppe der allgemeinen Formel $NR_3R_4$, in der $R_3$ und $R_4$ unabhängig voneinander jeweils ein Wasserstoffatom, eine geradkettige oder gegebenenfalls verzweigte $(C_1-C_4)$-Alkylgruppe, eine $(C_3-C_6)$-Cycloalkylgruppe, eine $(C_3-C_6)$-Cycloalkylmethylgruppe, eine Phenylgruppe, eine Phenylmethylgruppe oder eine Pyridinylgruppe darstellen, oder $R_3$ und $R_4$ gemeinsam mit dem sie tragenden Stickstoffatom einen Pyrrolidin- oder Piperidinring bilden, und

n die Zahl 1, 2 oder 3 bedeuten,

in Form der freien Base oder eines Säureadditionssalzes.

2. Verbindung nach Anspruch 1, **dadurch gekennzeichnet, daß** $R_1$ eine Methyl-, Ethyl- oder Phenylmethylgruppe, die gegebenenfalls am Phenylkern substituiert ist, $R_2$ eine Aminogruppe oder $(C_1-C_4)$-Alkylaminogruppe und n 1 bedeuten.

3. Verbindung nach Anspruch 1, **dadurch gekennzeichnet, daß** $R_1$ eine Phenylmethylgruppe, $R_2$ eine Aminogruppe und n 1 bedeuten.

4. Verbindung nach Anspruch 1, **dadurch gekennzeichnet, daß** $R_1$ eine Methylgruppe, $R_2$ eine Methylaminogruppe und n 1 bedeuten.

5. Verfahren zur Herstellung der Verbindungen nach Anspruch 1, **dadurch gekennzeichnet, daß** man ein Amin der allgemeinen Formel (II)

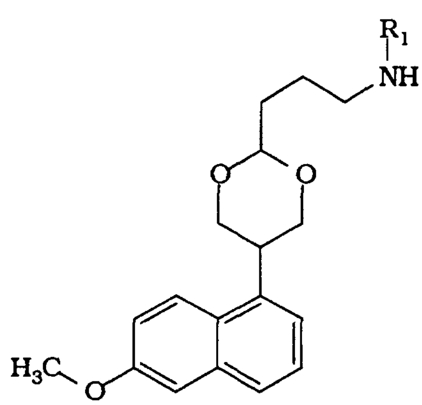

(II)

in der $R_1$ ein Wasserstoffatom oder eine Alkylgruppe darstellt, mit einem ω-Halogenalkanoat der allgemeinen

Formel (III)

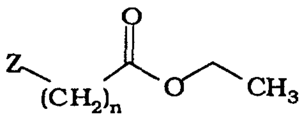

(III)

in der Z ein Chlor- oder Bromatom darstellt und n die in Anspruch 1 angegebene Bedeutung besitzt, umsetzt zur Bildung einer Verbindung der allgemeinen Formel (I), in der $R_1$ ein Wasserstoffatom oder eine Alkylgruppe und $R_2$ eine Ethoxygruppe bedeuten, wonach man gewünschtenfalls die in dieser Weise erhaltene Verbindung verseift, um sie in die entsprechende Säure umzuwandeln, oder man sie mit einem Amin der allgemeinen Formel $HNR_3R_4$, worin $R_3$ und $R_4$ die in Anspruch 1 angegebenen Bedeutungen besitzen, umsetzt, um die Verbindung in das Amid umzuwandeln.

6. Verfahren zur Herstellung der Verbindungen nach Anspruch 1, **dadurch gekennzeichnet, daß** man ein Amin der allgemeinen Formel (II)

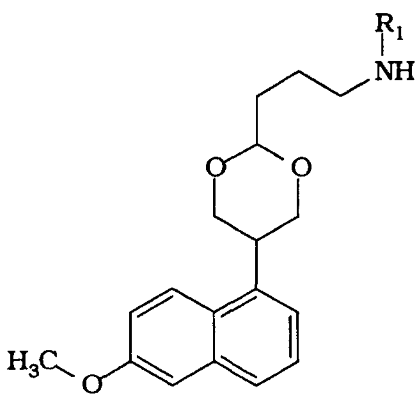

(II)

in der $R_1$ ein Wasserstoffatom oder eine Alkylgruppe darstellt, mit einem ω-Halogenalkanamid der allgemeinen Formel (IV)

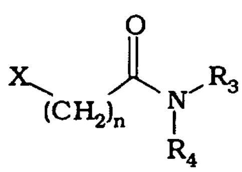

(IV)

in der X ein Chlor- oder Bromatom bedeutet und $R_3$, $R_4$ und n die in Anspruch 1 angegebenen Bedeutungen besitzen, umsetzt.

7. Verfahren zur Herstellung der Verbindungen nach Anspruch 1, **dadurch gekennzeichnet, daß** man ein Amin der allgemeinen Formel (II)

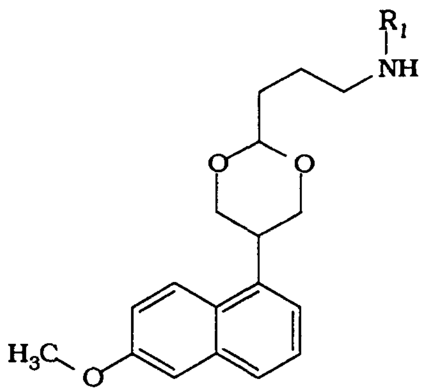

(II)

in der $R_1$ ein Wasserstoffatom oder eine Alkylgruppe darstellt, mit einem Propenamid der allgemeinen Formel (V)

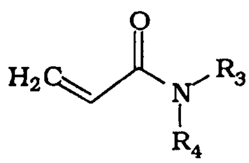

(V)

in der $R_3$ und $R_4$ die in Anspruch 1 angegebenen Bedeutungen besitzen, umsetzt.

**8.** Verfahren zur Herstellung der Verbindungen nach Anspruch 1, **dadurch gekennzeichnet, daß** man eine Alkylierung einer Verbindung der allgemeinen Formel (I), in der $R_1$ ein Wasserstoffatom bedeutet, durchführt.

**9.** Arzneimittel, **dadurch gekennzeichnet, daß** es aus einer Verbindung nach einem der Ansprüche 1 bis 4 besteht.

**10.** Pharmazeutische Zubereitung, **dadurch gekennzeichnet, daß** sie eine Verbindung nach einem der Ansprüche 1 bis 4 zusammen mit einem Trägermaterial enthält.

## Claims

**1.** Compound, in the form of a pure stereoisomer or a mixture of stereoisomers, corresponding to the general formula (I)

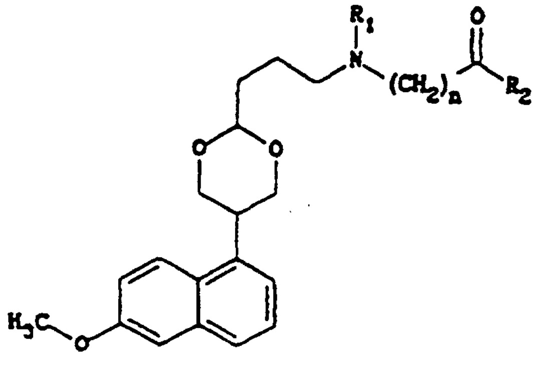

(I)

in which

$R_1$ represents a hydrogen atom or a $(C_1\text{-}C_4)$alkyl, $(C_3\text{-}C_6)$ cycloalkylmethyl or phenyl $(C_1\text{-}C_3)$ alkyl group optionally substituted on the phenyl ring with one or more atoms or groups chosen from halogens and methyl, trifluoromethyl, methoxy and cyano groups,

$R_2$ represents a hydroxyl or ($C_1$-$C_4$)alkoxy group or a group of general formula $NR_3R_4$ in which $R_3$ and $R_4$, independently of each other, each represent a hydrogen atom, a linear or optionally branched ($C_1$-$C_4$)alkyl group, a ($C_3$-$C_6$)cycloalkyl group, a ($C_3$-$C_6$)cycloalkylmethyl group, a phenyl group, a phenylmethyl group or a pyridyl group, or alternatively $R_3$ and $R_4$ form, together with the nitrogen atom which bears them, a pyrrolidine or piperidine ring, and

n represents the number 1, 2 or 3,

in the form of the free base or an addition salt with an acid.

**2.** Compound according to Claim 1, **characterized in that** $R_1$ represents a methyl, ethyl or phenylmethyl group optionally substituted on the phenyl ring, $R_2$ represents an amino or ($C_1$-$C_4$)alkylamino group and n is equal to 1.

**3.** Compound according to Claim 1, **characterized in that** $R_1$ represents a phenylmethyl group, $R_2$ represents an amino group and n is equal to 1.

**4.** Compound according to Claim 1, **characterized in that** $R_1$ represents a methyl group, $R_2$ represents a methylamino group and n is equal to 1.

**5.** Process for the preparation of compounds according to Claim 1, **characterized in that** an amine of general formula (II)

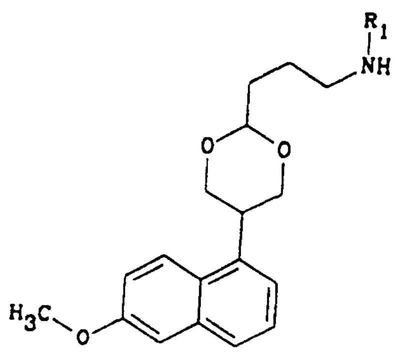

(II)

in which $R_1$ represents a hydrogen atom or an alkyl group, is reacted with an ω-halo alkanoate of general formula (III)

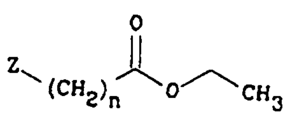

(III)

in which Z represents a chlorine or bromine atom and n is as defined in Claim 1, to obtain a compound of general formula (I) in which $R_1$ represents a hydrogen atom or an alkyl group and $R_2$ represents an ethoxy group, then, if so desired, the compound thus obtained is saponified to convert it into the corresponding acid, or alternatively it is reacted with an amine of general formula $HNR_3R_4$, in which $R_3$ and $R_4$ are as defined in Claim 1, to convert it into amide.

**6.** Process for the preparation of compounds according to Claim 1, **characterized in that** an amine of general formula (II)

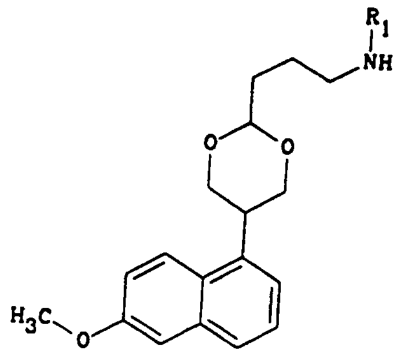

(II)

in which $R_1$ represents a hydrogen atom or an alkyl group, is reacted with an ω-halo alkanamide of general formula (IV)

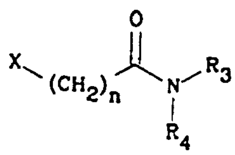

(IV)

in which X represents a chlorine or bromine atom and $R_3$, $R_4$ and n are as defined in Claim 1.

7. Process for the preparation of compounds according to Claim 1, **characterized in that** an amine of general formula (II)

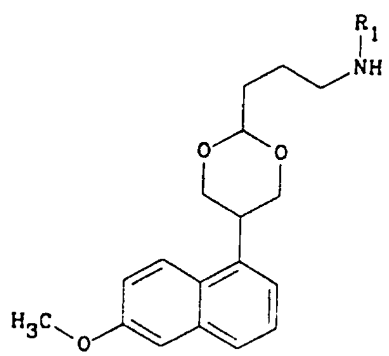

(II)

in which $R_1$ represents a hydrogen atom or an alkyl group, is reacted with a propenamide of general formula (V)

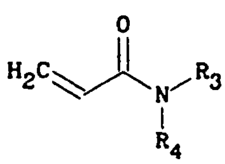

(V)

in which $R_3$ and $R_4$ are as defined in Claim 1.

8. Process for the preparation of compounds according to Claim 1, **characterized in that** an alkylation is carried out on a compound of general formula (I) in which $R_1$ represents a hydrogen atom.

9. Medicament, **characterized in that** it consists of a compound according to one of Claims 1 to 4.

**10.** Pharmaceutical composition, **characterized in that** it contains a compound according to one of Claims 1 to 4, combined with an excipient.